# EUROPEAN PATENT APPLICATION

(11) **EP 1 852 096 A1**
(43) Date of publication of application: **07.11.2007**
(21) Application number: 07106968.6
(22) Date of filing: 25.04.2007
(51) Int. Cl.: A61F 9/007

(54) **Phacoemulsification tip**

(30) Priority: 05.05.2006 US 418610
(71) Applicant: Alcon, Inc., 6331 Hünenberg (CH)
(72) Inventor: Boukhny, Mikhail, Laguna Niguel, CA 92677 (US)
(74) Representative: Hanna, Peter William Derek

(57) **Abstract**

A phacoemulsification tip is provided comprising a shaft (400) adapted to be mounted on a surgical handpiece (9), comprising a tubular shaft portion (412) having a longitudinal centreline (415) and terminating in a distal end portion (413), wherein the distal end portion lies on a line (417) that is offset from, but generally parallel to, the centreline of the shaft portion, for example when viewed from above or below. Such a construction provides a whipping motion when the tip is vibrated longitudinally.

## Description

### Background of the Invention

This invention relates generally to the field of phacoemulsification and more particularly to phacoemulsification cutting tips.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an IOL.

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

One phacoemulsification tip that has gained widespread acceptance has a belled or flared distal end. Such a tip is described in U.S. Patent No. 4,816,018 (Parisi). Such a design allows for larger lens material purchase as well as increased holding force when vacuum is applied to the tip while maintaining a smaller bore in the shaft of the tip. This combination of features increases anterior chamber stability, by reducing sudden outflow from the anterior chamber when the distal end becomes occluded and this occlusion breaks.

Another phacoemulsification tip is an angled or "bent" tip with or without a flared distal end. These tips are described in U.S. Patent No. 6,039,715 (Mackool), U.S. Patent No. 5,653,724 (Imonti) and U.S. Patent No. 5,154,694 (Kelman). These tips have a predominantly straight shaft with the far distal portion of the shaft being bent on an angle. Bent tips are used by a great many surgeons, and are particularly useful when used in conjunction with a oscillatory phacoemulsification handpiece, such as those described in U.S. Patent No. 6,352,519 (Anis, et al.) and U.S. Patent No. 6,602,193 (Chon) and commercially available as the NeoSoniX^{®} handpiece from Alcon Laboratories, Inc., Fort Worth, Texas, however; some surgeons are reluctant they feel that due to the proximal location of the bend it is more difficult to judge the position of the proximal cutting edge based on the extrapolation of the sleeved portion of the tip.

Angled phacoemulsification tips are particularly advantageous when used in combination with torsional ultrasound handpiece. Torsional ultrasound handpieces are more fully disclosed in U.S. Patent No. 6,077,285 (Boukhny). Torsional handpieces twist the tip, and cause a whipping motion in the tip when used with an angled tip. Producing such torsional movement in a tip requires the use of a special torsional handpiece, and the more commonly available handpieces produce only longitudinal motion along the centerline of the shaft.

Therefore, a need continues to exist for a phacoemulsification tip that produces a whipping motion when vibrated longitudinally.

### Brief Summary of the Invention

The present invention improves upon the prior art by providing a phacoemulsification tip containing a shaft that, when observed from above or below, contains a distal end is offset relative to the centerline the shaft. Such a construction produces a whipping motion when the tip is vibrated longitudinally.

Accordingly, one objective of the present invention is to provide a phacoemulsification cutting tip having increased efficiency.

Another objective of the present invention is to provide a phacoemulsification cutting tip having a shaft that, when observed from above or below, contains a distal end is offset relative to the centerline the shaft.

Another objective of the present invention is to provide a phacoemulsification cutting tip having a shaft that produces a whipping motion when the tip is vibrated longitudinally.

These and other advantages and objectives of the present invention will become apparent from the detailed description and claims that follow.

### Brief Description of the Drawings

FIG. 1 is a perspective view of a handpiece and control console that may be used with the present invention.
FIG. 2 is a perspective view of the distal end of a typical prior art straight shaft phacoemulsification tip.
FIG. 3 is an elevational view the distal end of a typical prior art angled or bent phacoemulsification tip.
FIG. 4 is a plan view the phacoemulsification tip of the present invention.
FIG. 5 is an elevational view the phacoemulsification tip of the present invention.

### Detailed Description of the Invention

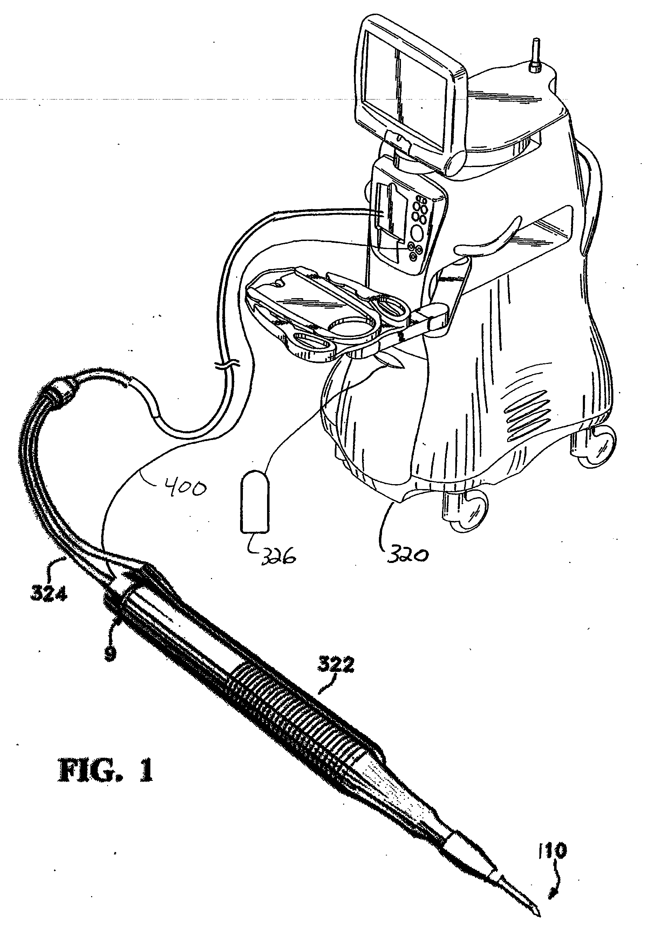

As best seen in FIG. 1, surgical console 320 suitable for use with the present invention may be any commercially available surgical control console such as the INFINITI^{®} surgical systems available from Alcon Laboratories, Inc., Fort Worth, Texas. Console 320 is connected to handpiece 9 through irrigation line 322 and aspiration line 324, and the flow through lines 322 and 324 is controlled by the user, for example, via footswitch 326. Power is supplied to handpiece through electrical cable 400.

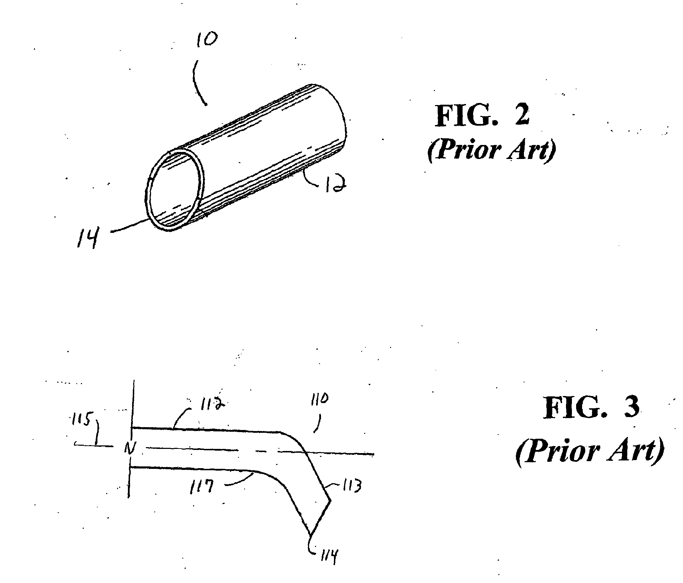

As best seen in FIG. 2, prior art phacoemulsification tip 10 contains shaft 12 that is straight all the way to distal tip 14. As best seen in FIG. 3, prior art phacoemulsification tip 110 contains shaft 112 that is straight up to distal end 113. When observed from the side, or elevational view, distal end 113 is angled or bent on an angle relative to centerline 115 of shaft 112 from intersection 117 of shaft 112 and distal end 113 all the way to distal tip 114.

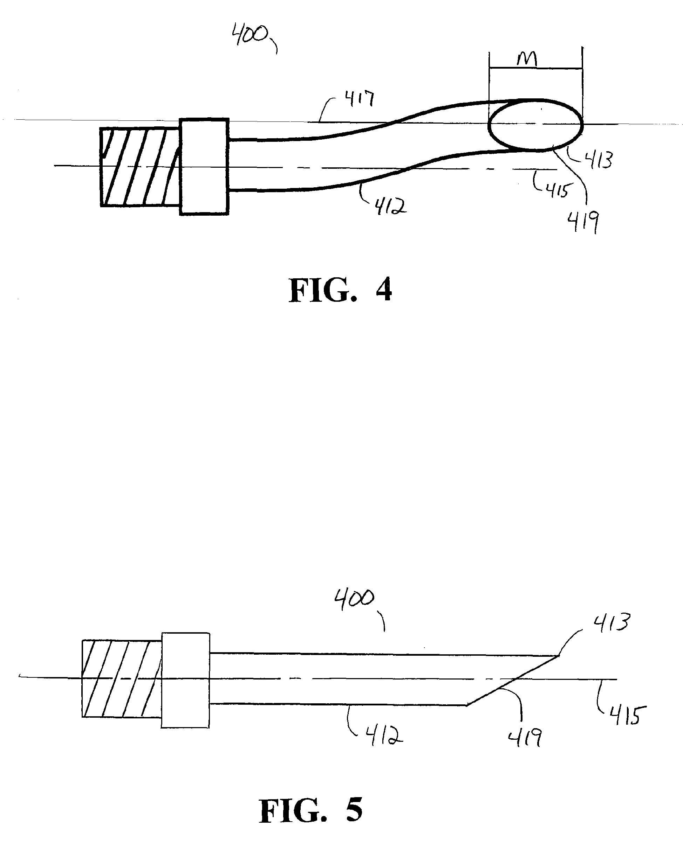

As best seen in FIGS. 4 and 5, tip 400 of the present invention contains shaft 412 having opening 419 that generally is cut at an angle relative to longitudinal centerline 415 forming oval or elliptical opening 419. Shaft 412, when observed from above or below, contains distal end 413 is offset relative to centerline 415 of shaft 412 so that distal tip 413 lies on line 417 that runs through major diameter M of opening 419 and is offset from, but generally parallel to, centerline 415. Such a construction produces a whipping motion when the tip is vibrated longitudinally.

This description is given for purposes of illustration and explanation. It will be apparent to those skilled in the relevant art that changes and modifications may be made to the invention described above without departing from its scope or spirit.

## Claims

1. A phacoemulsification tip (400) adapted to be mounted on a surgical handpiece (9), comprising a tubular shaft portion (412) having a longitudinal centreline (415) and terminating in a distal end portion (413),
**characterized in that** the distal end portion lies on a line (417) that is offset from, but generally parallel to, the centreline of the shaft portion.

2. The phacoemulsification tip of claim 1, wherein the distal end portion lies on a line (417) that is offset from, but generally parallel to, the longitudinal centreline (415) of the shaft portion (412), when observed from above or below.

3. The phacoemulsification tip of claim 1 or claim 2, wherein the distal end portion (413) defines an opening (419) cut at an angle relative to the longitudinal centreline (415) of the shaft (412) forming an oval or elliptical opening.

4. The phacoemulsification tip of claim 3, wherein the major diameter (M) of the oval or elliptical opening (419) is on a line (417) that is offset from, but generally parallel to, the longitudinal centreline (415) of the shaft portion (412).
